# FASCICULE DE BREVET EUROPEEN

(11) **EP 0 666 857 B1**
(45) Date de publication et mention de la délivrance du brevet: **19.05.1999**
(21) Numéro de dépôt: 93924646.8
(22) Date de dépôt: 28.10.1993
(51) Int. Cl.: C07D 413/12, C07D 305/14, C07D 263/04

(54) **PROCEDE DE PREPARATION DE DERIVES DU TAXANE**
VERFAHREN ZUR HERSTELLUNG VON TAXANDERIVATEN
METHOD FOR PREPARING TAXANE DERIVATIVES

(30) Priorité: 30.10.1992 FR 9213000
(43) Date de publication de la demande: 16.08.1995
(73) Titulaire: RHONE-POULENC RORER S.A., 92160 Antony (FR)
(72) Inventeur: DENIS, Jean-Noel, Apt. No. 3, Le Pinet, F-38410 Uriage (FR); GREENE, Andrew, Saint-Martin-d'Uriage, F-38410 Uriage (FR); KANAZAWA, Alice, F-38000 Grenoble (FR)
(74) Mandataire: Pilard, Jacques
(86) Numéro de dépôt international: FR9301058
(87) Numéro de publication internationale: WO9410169

(56) Documents cités:
- WO-A-92/09589
- WO-A-94/07877
- WO-A-94/07878
- TETRAHEDRON LETTERS, vol. 33, no. 36 , 1 Septembre 1992 Oxford, GB, pages 5185 - 5188 A. COMMERCON, ET AL.: 'Improved protection and esterification of a precursor of the Taxotere and taxol side chains'

## Description

La présente invention concerne un procédé de préparation de dérivés du taxane de formule générale : 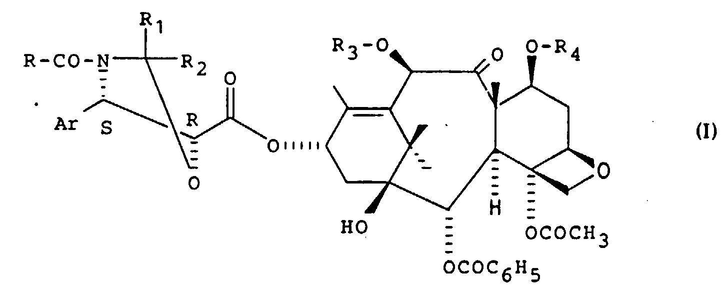 qui sont des intermédiaires particulièrement intéressants pour préparer le taxol, le Taxotère et leurs analogues qui présentent des propriétés antitumorales et antileucémiques remarquables.

Dans la formule générale (I),
Ar représente un radical aryle,
R représente le radical phényle ou un radical R₅-O- dans lequel R₅ représente
   - un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle cyano, carboxy ou alcoxycarbonyle dont la partie alcoyle contient 1 à 4 atomes de carbone,
   - ou un radical phényle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone,
   - un radical hétérocyclyle azoté saturé ou non saturé contenant 4 à 6 chaînons et éventuellement substitué par un ou plusieurs radicaux alcoyles contenant 1 à 4 atomes de carbone, étant entendu que les radicaux cycloalcoyles, cycloalcényles ou bicycloalcoyles peuvent être éventuellement substitués par un ou plusieurs radicaux alcoyles contenant 1 à 4 atomes de carbone,
R₁ et R₂, identiques ou différents, représentent un atome d'hydrogène, un radical alcoyle, phénylalcoyle, phényle, alcoxyphényle ou dialcoxyphényle, ou bien R₁ et R₂ forment ensemble avec l'atome de carbone auquel ils sont liés un cycle ayant de 4 à 7 chaînons,
R₃ représente un radical acétyle ou un groupement protecteur de la fonction hydroxy et
R₄ représente un groupement protecteur de la fonction hydroxy.

Plus particulièrement, Ar représente un radical phényle ou α- ou β-naphtyle éventuellement substitué par un ou plusieurs atomes ou radicaux, identiques ou différents, choisis parmi les atomes d'halogène (fluor, chlore, brome, iode) et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et que les radicaux aryles sont des radicaux phényles ou α- ou β-naphtyles, ou bien Ar représente un radical hétérocyclique aromatique ayant 5 chaînons et contenant un ou plusieurs atomes, identiques ou différents, choisis parmi les atomes d'azote, d'oxygène ou de soufre, éventuellement substitué par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

Plus particulièrement, Ar représente un radical phényle, thiényl-2 ou -3 ou furyle-2 ou -3 éventuellement substitué par un ou plusieurs atomes ou radicaux, identiques ou différents, choisis parmi les atomes d'halogène et les radicaux alcoyles, alcoxy, amino, dialcoylamino, acylamino, alcoxycarbonylamino et trifluorométhyle.

Plus particulièrement encore, Ar représente un radical phényle éventuellement substitué par un atome de chlore ou de fluor, ou par un radical alcoyle (méthyle), alcoxy (méthoxy), dialcoylamino (diméthylamino), acylamino (acétylamino) ou alcoxycarbonylamino (t.butoxycarbonylamino) ou thiényle-2 ou -3 ou furyle-2 ou -3.

Plus particulièrement, R₃ représente un radical acétyle ou un groupement protecteur de la fonction hydroxy choisi parmi les radicaux (2,2,2-trichloroéthoxy) carbonyle, (2-trichlorométhylisopropoxy) carbonyle, trialcoylsilyles, dialcoylarylsilyles, alcoyldiarylsilyles ou triarylsilyles dans lesquels les radicaux alcoyles contiennent 1 à 4 atomes de carbone et les radicaux aryles sont, de préférence, des radicaux phényles et R4 représente un groupement protecteur de la fonction hydroxy choisi parmi les radicaux (2,2,2-trichloroéthoxy) carbonyle, (2-trichlorométhylisopropoxy) carbonyle, benzyle, 4-méthoxybenzyle, 2,4-diméthoxybenzyle, trialcoylsilyles, dialcoylarylsilyles, alcoyldiarylsilyles ou triarylsilyles dans lesquels les radicaux alcoyles contiennent 1 à 4 atomes de carbone et les radicaux aryles sont, de préférence, des radicaux phényles.

Dans la demande internationale PCT WO 9209589 est décrite la préparation des produits de formule générale (I) par estérification de la baccatine III protégée ou de la 10-désacétylbaccatine III protégée de formule générale : 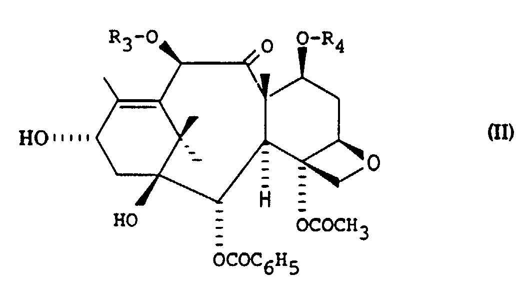 dans laquelle R₃ et R₄ sont définis comme précédemment, au moyen d'un acide de formule générale : 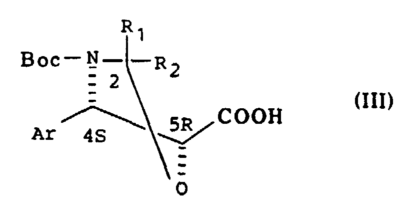 dans laquelle Ar, R₁ et R₂ sont définis comme précédemment et Boc représente le radical t.butoxycarbonyle, et leur transformation en taxol, Taxotère ou leurs dérivés de formule générale: 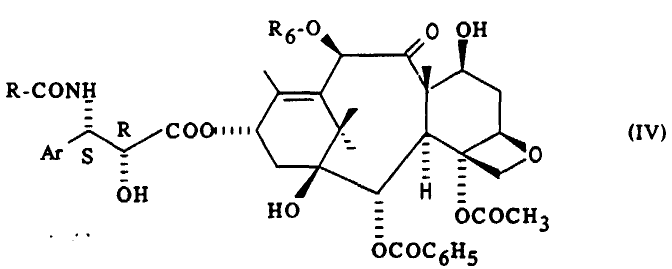 dans laquelle Ar est défini comme précédemment, R₆ représente un atome d'hydrogène ou le radical acétyle et R représente le radical phényle ou un radical R₅-O- dans lequel R₅ est défini comme précédemment, en passant intermédiairement par un produit de formule générale : 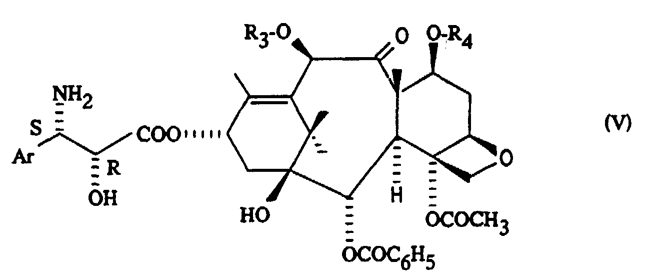 dans laquelle R₃ et R₄ sont définis comme précédemment.

Selon les procédés antérieurement connus, il était nécessaire, pour obtenir les produits de formule générale (IV) dont la configuration absolue de la chaîne latérale est nécessaire à l'activité antitumorale, d'utiliser l'acide de formule générale (m) dont les atomes de carbone en -4 et -5 ont respectivement les configurations S et R.

Il a maintenant été trouvé, et c'est ce qui fait l'objet de la présente invention que les produits de formule générale (I) peuvent être obtenus, avec une stéréosélectivité voisine de 100 %, par estérification de la baccatine III protégée ou de la 10-désacétylbaccatine III protégée au moyen d'un acide de formule générale : 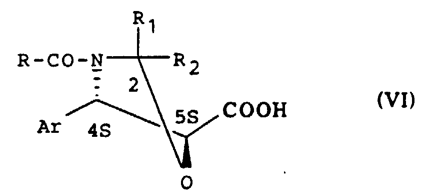 dans laquelle Ar, R, R₁ et R₂ sont définis comme précédemment, et dont les atomes de carbone en position -4 et -5 ont chacun la configuration S, ou d'un dérivé activé de cet acide.

Le procédé selon l'invention permet d'obtenir stéréosélectivement le produit de formule générale (I) à partir d'un acide de formule générale (VI) éventuellement en mélange avec un acide de formule générale (III).

Selon la présente invention, l'estérification de la baccatine III protégée ou de la 10-désacétylbaccatine III protégée par l'acide de formule générale (VI) est effectuée en présence d'un agent de condensation tel qu'un imide comme le dicyclohexylcarbodiimide ou un carbonate réactif comme le 2-dipyridylcarbonate et d'un agent d'activation tel qu'une aminopyridine comme la 4-diméthylaminopyridine ou la 4-pyrrolidinopyridine en opérant dans un solvant organique choisi parmi les éthers tels que le tétrahydrofuranne, le diisopropyléther, le méthyl t.butyléther ou le dioxanne, les cétones telles que la méthylisobutylcétone, les esters tels que l'acétate d'éthyle, l'acétate d'isopropyle ou l'acétate de n.butyle, les nitriles, les hydrocarbures aliphatiques tels que le pentane, l'hexane ou l'heptane, les hydrocarbures aliphatiques halogénés tels que le dichlorométhane ou le 1,2-dichloroéthane et les hydrocarbures aromatiques tels que le benzène, le toluène, les xylènes, l'éthylbenzène, l'isoropylbenzène ou le chlorobenzène, à une température comprise entre 0 et 90°C.

L'estérification peut aussi être réalisée en utilisant l'acide de formule générale (VI) sous forme d'anhydride de formule générale : 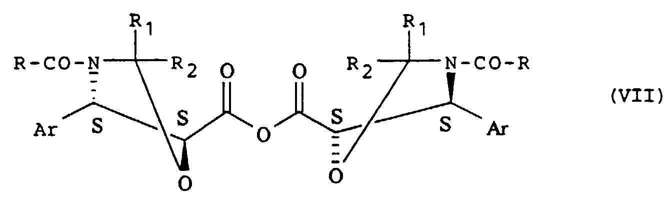 dans laquelle Ar, R, R₁ et R₂ sont définis comme précédemment, en présence d'un agent d'activation tel qu'une aminopyridine comme la 4-diméthylaminopyridine ou la 4-pyrrolidinopyridine en opérant dans un solvant organique choisi parmi les éthers tels que le tétrahydrofuranne, le diisopropyléther, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le 1,2-dichloroéthane et les hydrocarbures aromatiques tels que le benzène, le toluène, les xylènes, l'éthylbenzène, l'isopropylbenzène ou le chlorobenzène à une température comprise entre 0 et 90°C.

L'estérification peut aussi être réalisée en utilisant l'acide de formule générale (VI) sous forme d'halogénure ou d'anhydride mixte de formule générale : 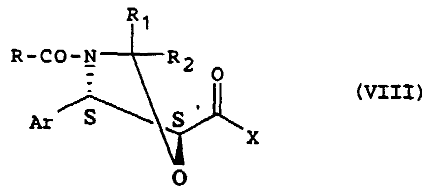 dans laquelle Ar, R, R₁ et R₂ sont définis comme précédemment et X représente un atome d'halogène ou un radical acyloxy ou aroyloxy, éventuellement préparé in situ, en présence d'une base qui est de préférence une base organique azotée telle qu'une amine aliphatique tertiaire, une pyridine ou une aminopyridine comme la diméthylamino-4 pyridine ou la pyrrolidino-4 pyridine en opérant dans un solvant organique inerte choisi parmi les éthers tels que le tétrahydrofuranne, le diisopropyléther, le méthyl t.butyléther ou le dioxanne, les cétones comme la méthyl t.butylcétone, les esters comme l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le 1,2-dichloroéthane et les hydrocarbures aromatiques tels que le benzène, le toluène, les xylènes, l'éthylbenzène, l'isopropylbenzène ou le chlorobenzène à une température comprise entre 0 et 90°C.

L'acide de formule générale (VI) peut être obtenu par saponification de l'ester de formule générale : 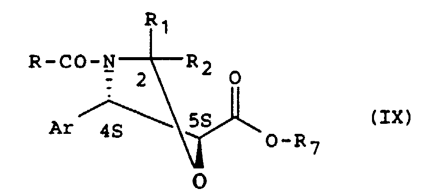 dans laquelle Ar, R, R₁ et R₂ sont définis comme précédemment et R₇ représente un radical alcoyle contenant 1 à 4 atomes de carbone éventuellement substitué par un ou plusieurs radicaux phényles.

Généralement, la saponification est effectuée en milieu aqueux, éventuellement en présence d'un alcool aliphatique contenant 1 à 4 atomes de carbone (méthanol, éthanol, isopropanol, t.butanol), en présence d'une base minérale choisie parmi les hydroxydes, les carbonates ou les bicarbonates de métaux alcalins ou alcalino-terreux, à une température comprise entre 0 et 50°C, de préférence voisine de 20°C.

L'ester de formule générale (IX) peut être obtenu par action d'un aldéhyde ou d'une cétone de formule générale: 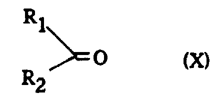 dans laquelle R₁ et R₂ sont définis comme précédemment, éventuellement sous forme d'un dialcoylacétal ou d'un alcoyléther d'énol, sur un ester de formule générale : 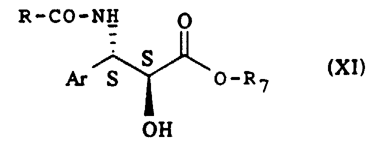 dans laquelle Ar, R et R₇ sont définis comme précédemment, en opérant dans un solvant organique inerte en présence d'un acide fort minéral, tel que l'acide sulfurique, ou organique, tel que l'acide p.toluènesulfonique éventuellement sous forme de sel de pyridinium à une température comprise entre 0° et la température d'ébullition du mélange réactionnel. Les solvants qui conviennent particulièrement bien sont les hydrocarbures aromatiques tels que le toluène.

L'ester de formule générale (XI) peut être obtenu par action du chlorure de benzoyle ou d'un produit de formule générale R₅-O-CO-Y dans laquelle R₅ est défini comme précédemment et Y représente un atome d'halogène ou un radical -O-R₅ ou -O-CO-R₅ sur un ester de formule générale : 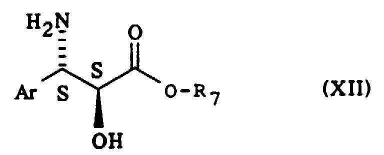 dans laquelle Ar et R₇ sont définis comme précédemment, en opérant dans un solvant organique tel qu'un ester aliphatique comme l'acétate d'éthyle ou un hydrocarbure aliphatique halogéné comme le dichlorométhane éventuellement en présence d'une base minérale telle que le bicarbonate de sodium ou organique telle que la triéthylamine. Généralement, la réaction est effectuée à une température comprise entre 0 et 50°C, de préférence voisine de 20°C.

L'ester de formule générale (XII) peut être obtenu selon le procédé décrit par E. Kamandi et coll., Arch. Pharmaz., 308, 135-141 (1975).

L'anhydride de formule générale (VII) peut être obtenu en faisant réagir un agent de déshydratation tel que le dicyclohexylcarbodiimide sur l'acide de formule générale (VI) en opérant dans un solvant organique choisi parmi les éthers tels que le tétrahydrofuranne, le diisopropyléther, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène, les xylènes, l'éthylbenzène, l'isopropylbenzène ou le chlorobenzène à une température comprise entre 0 et 30°C.

L'acide activé de formule générale (VIII) peut être obtenu par action d'un halogénure de sulfuryle, de préférence le chlorure, ou d'un produit de formule générale :

R₈-CO-Z (XIII)

dans laquelle R₈ représente un radical alcoyle contenant 1 à 4 atomes de carbone ou un radical phényle éventuellement substitué par 1 à 5 atomes ou radicaux, identiques ou différents, choisis parmi les atomes d'halogène et les radicaux nitro, méthyle ou méthoxy et Z représente un atome d'halogène, de préférence un atome de chlore, sur un acide de formule générale (VI) en opérant dans un solvant organique convenable tel que le tétrahydrofuranne en présence d'une base organique telle qu'une amine tertiaire comme la triéthylamine à une température comprise entre 0 et 30°C.

L'acide de formule générale (VI) peut aussi être obtenu par oxydation d'un produit de formule générale : 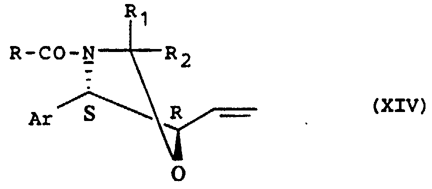 dans laquelle Ar, R, R₁ et R₂ sont définis comme précédemment.

Généralement, l'oxydation est effectuée au moyen d'un periodate alcalin (periodate de sodium) en présence d'une quantité catalytique d'un sel de ruthénium (RuCl₃) et de bicarbonate de sodium en opérant en milieu hydro-organique tel que par exemple un mélange acétonitrile-tétrachlorure de carbone-eau. Généralement, la réaction est effectuée à une température voisine de 20°C.

Le produit de formule générale (XIV) peut être obtenu par action d'un aldéhyde ou d'une cétone de formule générale (X), éventuellement sous forme d'un dialcoylacétal ou d'un ester d'énol, sur unproduit de formule générale : 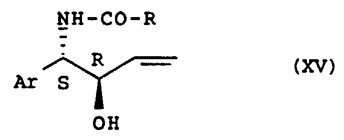 dans laquelle Ar et R sont définis comme précédemment, en opérant dans un solvant organique inerte en présence d'un acide fort minéral, tel que l'acide sulfurique, ou organique, tel que l'acide p.toluènesulfonique éventuellement sous forme de sel de pyridinium, à une température comprise entre 0°C et la température d'ébullition du mélange réactionnel. Les solvants qui conviennent particulièrement bien sont les hydrocarbures aromatiques.

Le produit de formule générale (XV) peut être obtenu dans les conditions décrites dans EP-A- 0 530 385.

L'ester de formule générale (IX) dans laquelle Ar et R₇ sont définis comme précédemment, R₁ représente un atome d'hydrogène et, R₂ représente un radical phényle, alcoxyphényle ou dialcoxyphényle, peut aussi être obtenu par cyclisation d'un produit de formule générale: 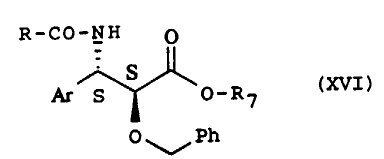 dans laquelle Ar, R et R₇ sont définis comme précédemment et Ph représente un radical phényle, alcoxyphényle ou dialcoxyphényle, en opérant de préférence en milieu anhydre, dans un solvant organique choisi parmi les éthers, les esters, les cétones, les nitriles, les hydrocarbures aliphatiques éventuellement halogénés et les hydrocarbures aromatiques éventuellement halogénés en présence d'un agent d'oxydation tel que la dichlorodicyanobenzoquinone à une température comprise entre 0°C et la température d'ébullition du mélange réactionnel. De préférence, on opère dans un hydrocarbure aliphatique halogéné, tel que le dichlorométhane, ou l'acétonitrile à une température voisine de 20°C.

La cyclisation conduit à la formation d'un mélange des épimères 2R et 2S du produit de formule générale (IX) qui peuvent être séparés selon les méthodes habituelles. Il est particulièrement avantageux d'obtenir préférentiellement l'épimère 2R pour préparer le taxol, le Taxotère ou leurs dérivés à partir d'un produit de formule générale (I).

L'invention concerne également les acides de formule générale (VI) éventuellement sous forme de sel, d'ester, d'anhydride, d'anhydride mixte ou d'halogénure, et les dérivés de formule I dans laquelle
Ar représente un radical aryle,
R représente le radical phényle
R₁ et R₂, identiques ou différents, représentent un atome d'hydrogène, un radical alcoyle, phénylalcoyle, phényle, alcoxyphényle ou dialcoxyphényle, ou bien R1 et R2 forment ensemble avec l'atome de carbone auquel ils sont liés un cycle ayant de 4 à 7 chaînons, étant entendu que, lorsque l'un des R₁ ou R₂ représente un atome d'hydrogène, alors l'autre des R₁ ou R₂ ne représente ni un atome d'hydrogène, ni un radical aryle éventuellement substitué,
R₃ représente un radical acétyle ou un groupement protecteur de la fonction hydroxy et
R₄ représente un groupement protecteur de la fonction hydroxy.

WO-A-9407878 a été publié le 14.04.94 et est un état de la technique selon l'article 54(3) de la CBE. Ce document divulgue les dérivés de formule I dans laquelle l'un des R₁ ou R₂ représente un atome d'hydrogène et l'autre des R₁ ou R₂ représente un atome d'hydrogène ou un radical aryle éventuellement substitué.

Les dérivés du taxane de formule générale (I) obtenus par la mise en oeuvre du procédé selon l'invention peuvent être transformés en taxol, en Taxotère ou en leurs analogues selon les procédés décrits dans la demande internationale PCT WO 9209589, lorsque R₁ et R₂ représentent chacun un radical alcoyle ou phénylalcoyle en passant intermédiairement par le produit de formule générale (V) ou bien par traitement en milieu acide (acide chlorhydrique, acide sulfurique, acide acétique, acide méthanesulfonique, acide trifluorométhanesulfonique, acide p.toluènesulfonique) en opérant dans un solvant organique (alcool, éther, ester, hydrocarbure aliphatique, hydrocarbure aliphatique halogéné, hydrocarbure aromatique, nitrile) à une température comprise entre -10 et 60°C, lorsque R₁ représente un atome d'hydrogène et R₂ représente un radical phényle, alcoxyphényle ou dialcoxyphényle en passant intermédiairement par un produit de formule générale 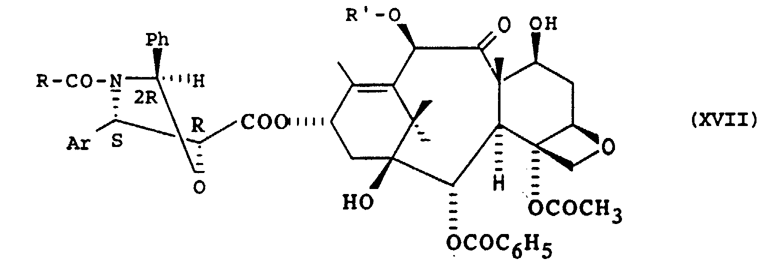 dans laquelle Ar, R et Ph sont définis comme précédemment et R' représente un atome d'hydrogène ou le radical acétyle, après remplacement des groupements protecteurs R₄ et éventuellement R₃ par des atomes d'hydrogène selon les méthodes connues.

Les exemples suivants illustrent la présente invention.

### EXEMPLE 1

Dans un ballon de 10 cm3, muni d'un système d'agitation magnétique, on met, sous atmosphère d'argon, 28 mg (0,087 mmole) d'acide tbutoxycarbonyl-3 diméthyl-2,2 phényl4 oxazolidine-1,3 carboxylique-5-(4S,5S) en solution dans 1,5 cm3 de toluène anhydre. On ajoute ensuite 18 mg (0,087 mmole) de dicyclohexylcarbodiimide distillé. On laisse réagir pendant 5 minutes à une température voisine de 20°C puis on ajoute, en une seule fois, un mélange de 3,5 mg (0,029 mmole) de N,N-diméthylamino-4 pyridine et 26 mg (0,029 mmole) d'acétoxy-4 benzoyloxy-2α époxy-5β,20 dihydroxy-1,13α oxo-9 bis-(trichloro-2,2,2 éthoxycarbonyloxy)-7β,10β taxène-11. On laisse réagir pendant 5 minutes à une température voisine de 20°C puis on chauffe pendant 16 heures à 72°C. Après refroidissement à une température voisine de 20°C, on ajoute 40 cm3 d'acétate d'éthyle. La phase organique est lavée avec 5 cm3 d'eau distillée, 2 fois 5 cm3 d'une solution aqueuse saturée d'hydrogénocarbonate de sodium puis avec 5 cm3 d'une solution aqueuse saturée de chlorure de sodium, et enfin séchée sur sulfate de sodium anhydre. Après filtration et évaporation des solvants sous pression réduite, on obtient un résidu (solide) qui est purifié par chromatographie préparative sur couche mince de silice en éluant avec un mélange éther-hexane-dichlorométhane (5-20-75 en volumes). On obtient ainsi, avec un rendement de 86 %, 30 mg (0,025 mmole) de t.butoxycarbonyl-3 diméthyl-2,2 phényl-4 oxazolidine-1,3-carboxylate-5-(4S,5R) d'acétoxy-4 benzoyloxy-2α époxy-5β,20 hydroxy-1 oxo-9 bis-(trichloro-2,2,2 éthoxycarbonyl)oxy-7β,10β taxène-11 yle-13α dont les caractéristiques sont les suivantes :
- spectre infra-rouge (film) : principales bandes d'absorption caractéristiques à 3450, 2970, 2910, 1760, 1720, 1700, 1600, 1580, 1450, 1375, 1360, 1245, 1170, 1135, 1100, 1080, 1060, 1020, 995, 975, 960, 900, 820, 765 et 720 cm⁻¹
- spectre de résonance magnétique nucléaire du proton (300 MHz ; CDCl₃ ;: déplacements chimiques en ppm ; constantes de couplage J en Hz) : 1,18 (s, 12H) ; 1,27 (s, 3H) ; 1,6-2,0 (m, 1H) ; 1,76 (s, 3H) ; 1,81 (s, 3H) ; 1,83 (s, 3H) ; 1,95 (s, 3H) ; 2,05 (s, 3H) ; 2,20 (d, J = 9, 2H) ; 2,55-2,65 (m, 1H); 3,90 (d, J = 7, 1H); 4,20 (AB_{q}, J_{AB} = 8,5, δ_{A}-δ_{B} = 47,2, 2H) ; 4,47 (d, J = 6,4, 1H) ; 4,75 (AB_{q}, J_{AB} = 12, δ_{A}-δ_{B} = 92,2, 2H) ; 4,78 (s, 2H) ; 4,91 (d, J = 12, 1H); 5,12 (s large, 1H); 5,58 (dd, J = 7,1 et 10,6, 1H); 5,67 (d, J = 7, 1H); 6,25 (s, 1H); 6,28 (t, J = 9, 1H); 7,2-7,4 (m, 5H aromatiques) ; 7,47-7,52 (m, 2H aromatiques) ; 7,61-7,66 (m, 1H aromatique) ; 8,03-8,05 (m, 2H aromatiques)
- spectre de masse (FAB (+)-matrice NBA) : ion moléculaire (massif) : M⁺ (1198).

L'acide t.butoxycarbonyl-3 diméthyl-2,2 phényl-4 oxazolidine-1,3 carboxylique-5-(4S,5S) peut être préparé de la manière suivante:

Dans un ballon de 25 cm3, muni d'un système d'agitation magnétique, on introduit successivement 40 mg (0,12 mmole) de t.butoxycarbonyl-3 diméthyl-2,2 phényl-4 méthoxycarbonyl-5 oxazolidine-1,3-(4S,5S), 5 cm3 de méthanol, 2 cm3 d'eau distillée et 50 mg (0,36 mmole) de carbonate de potassium solide. Le mélange réactionnel est agité pendant 40 heures à une température voisine de 20°C puis on évapore le méthanol sous pression réduite. On ajoute 13 cm3 d'eau puis lave la phase aqueuse obtenue 3 fois avec 20 cm3 d'éther éthylique. La phase aqueuse basique est refroidie à 0°C puis est acidifiée, en présence de 20 cm3 de dichlorométhane et sous forte agitation, par addition d'une solution aqueuse d'acide chlorhydrique 2M. La phase organique est séparée par décantation puis la phase aqueuse est extraite 6 fois avec 30 cm3 de dichlorométhane. Les phases organiques réunies sont lavées 3 fois avec 5 cm3 d'eau distillée puis 1 fois avec 5 cm3 d'une solution aqueuse saturée de chlorure de sodium puis enfin séchées sur sulfate de magnésium anhydre. Après filtration et concentration à sec sous pression réduite, on obtient, avec un rendement de 99 %, 38 mg (0,12 mmole) d'acide t.butoxycarbonyl-3 diméthyl-2,2 phényl-4 oxazolidine-1,3 carboxylique-5-(4S,5S) dont les caractéristiques sont les suivantes :
- spectre infra-rouge (film) : principales bandes d'absorption caractéristiques à 3650-2200, 2970, 2920, 1760, 1740, 1700, 1470, 1450, 1370, 1250, 1215, 1165, 1135, 1110, 1065, 875 et 690 cm⁻¹.
- spectre de résonance magnétique nucléaire du proton (200 MHz ; CDCl₃ ; déplacements chimiques en ppm ; constantes de couplage J en Hz) : 1,20 (maj) et 1,43 (min) (2s larges, 9H); 1,64 (s, 3H) ; 1,94 (s, 3H) ; 3,0 (s très large, 1H) ; 4,97 (d déformé, J = 7, 1H); 5-5,25 (m, 1H); 7,2-7,4 (m, 5H aromatiques).
- spectre de masse (i.c., NH₃ + isobutane) : 339 (MH⁺), 322 (MH⁺-OH), 283, 266, 222, 206, 158, 124, 110.

Le t.butoxycarbonyl-3 diméthyl-2,2 phényl-4 méthoxycarbonyl-5 oxazolidine-1,3-(4S,5S) peut être préparé de la manière suivante :

Dans un ballon de 10 cm3, muni d'un système d'agitation magnétique, on introduit, sous atmosphère d'argon, 147,5 mg (0,50 mmole) de t.butoxycarbonylamino-3 phényl-3 hydroxy-2 propionate-(2S,3S) de méthyle et 2,5 cm3 de toluène anhydre. On ajoute ensuite à la suspension obtenue 10 grains de tamis moléculaire 4Å, 188,5 µl (144,2 mg, 2,0 mmoles) de méthoxy-2 propène et 12,5 mg (0,05 mmole) de p.toluènesulfonate de pyridinium. On laisse réagir pendant 1 heure à une température voisine de 20°C puis on chauffe à 120°C et laisse réagir à cette température pendant 2 heures. Le mélange réactionnel brun foncé résultant est refroidi à une température voisine de 20°C. On ajoute 60 cm3 de dichlorométhane. La phase organique est lavée avec 5 cm3 d'une solution aqueuse saturée d'hydrogénocarbonate de sodium, 3 fois avec 5 cm3 d'eau puis 1 fois avec 5 cm3 d'une solution aqueuse saturée de chlorure de sodium. La phase organique est séchée sur sulfate de sodium anhydre. Après filtration et concentration à sec sous pression réduite, on obtient un résidu qui est chromatographié sur une colonne de gel de silice en éluant avec un mélange éther éthylique-hexane (15-85 en volumes). On obtient, avec un rendement de 36 %, 60 mg (0,18 mmole) de t.butoxycarbonyl-3 diméthyl-2,2 phényl-4 méthoxycarbonyl-5 oxazolidine-1,3-(4S,5S) dont les caractéristiques sont les suivantes :
- spectre infra-rouge (film) : principales bandes d'absorption caractéristiques à 3060, 3025, 2975, 2920, 1775, 1740, 1700, 1490, 1480, 1450, 1440, 1365, 1250, 1210, 1165, 1110, 1070, 1050, 1030, 890, 760, 720 et 695 cm⁻¹.
- spectre de résonance magnétique nucléaire du proton (200 MHz ; CDCl₃ ; déplacements chimiques en ppm ; constantes de couplage J en Hz) : 1,20 (maj) et 1,43 (min) (2s larges, 9H) ; 1,63 (s, 3H) ; 1,90 (min) et 1,95 (maj) (2s larges, 3H) ; 3,32 (s, 3H) ; 4,95-5,20 (m, 1H) ; 4,97 (d déformé, J = 7, 1H); 7,15-7,40 (m, 5H aromatiques).
- spectre de masse (I.C., NH₃ + isobutane) : 353 (M+NH₄⁺) ; 336 (MH⁺) ; 320 (M⁺-CH₃) ; 297 ; 280 ; 239 ; 236 ; 222 ; 220.

Le t.butoxycarbonylamino-3 phényl-3 hydroxy-2 propionate-(2S,3S) de méthyle peut être préparé de la manière suivante :

A une suspension de 6,5 g de phénylglycidate-(2S,3R) d'α-méthylbenzylamine dans 20 cm3 de toluène et 10 cm3 d'eau, on ajoute, en 5 minutes, une solution aqueuse de soude 4N. Après 2 heures d'agitation à une température voisine de 20°C, on extrait la phase aqueuse séparée par 2 fois 7 cm3 de toluène. La phase aqueuse est introduite dans un autoclave. Après avoir ajouté 97,5 cm3 d'une solution aqueuse d'ammoniaque à 32 % (p/v) et 1,22 g de chlorure d'ammonium, l'autoclave est fermé puis chauffé, sous agitation, pendant 6 heures à 60°C (pression autogène de 3 bars). Après refroidissement à une température voisine de 20°C, on ajoute 6 cm3 d'une solution aqueuse de soude 4N. On agite pendant 30 minutes, puis on élimine l'ammoniac sous pression réduite puis on concentre à sec sous pression réduite (45 mm de mercure; 6 kPa) à 45°C. Le résidu obtenu est repris par 75 cm3 de méthanol. A la suspension obtenue, on ajoute en 35 minutes, à 20°C, une solution méthanolique d'acide sulfurique constituée de 4,83 g d'acide sulfurique concentré dans 20 cm3 de méthanol. Le mélange réactionnel est chauffé à 50°C pendant 3 heures 30 minutes. Après refroidissement à 0°C, on ajoute une solution de 27 g de carbonate de sodium dans 20 cm3 d'eau. Après cessation du dégagement de gaz carbonique, le mélange réactionnel est refroidi à 23°C en 30 minutes. On ajoute alors, en 30 minutes, une solution de 6,1 g de dicarbonate de di-t.butyle dans 7 cm3 de méthanol. On agite pendant 4 heures puis, après évaporation de 50 cm3 de méthanol, on ajoute 60 cm3 d'eau puis évapore la totalité du méthanol. Le produit qui précipite est séparé par filtration, lavé avec 2 fois 25 cm3 d'eau et séché à poids constant. On obtient ainsi, avec un rendement de 30 %, 2 g de t.butoxycarbonylamino-3 phényl-3 hydroxy-2 propionate-(2S,3S) de méthyle dont les caractéristiques sont les suivantes :
- point de fusion : 135,5-136°C.
- pouvoir rotatoire: [α]_{D}²⁵ = + 29,6° (c = 0,5; chloroforme)
- spectre infra-rouge (film) : principales bandes d'absorption caractéristiques à 3380, 3350, 3000, 2970, 2930, 1720, 1690, 1510, 1435, 1385, 1360, 1310, 1285, 1230, 1205, 1170, 1105, 1005, 860, 770, 750, 730 et 690 cm⁻¹.
- spectre de résonance magnétique nucléaire du proton (200MHz ; CDCl₃ ; déplacements chimiques en ppm ; constantes de couplage J en Hz) : 1,43 (s, 9H) ; 2,84 (d, J = 7, 1H); 3,71 (s, 3H) ; 4,60 (dd, J = 3,5 et 7, 1H); 5,10 (d déformé, J = 8, 1H); 7,20-7,37 (m, 5H aromatiques).
- spectre de résonance magnétique nucléaire du proton (360 MHz ; DMSO d₆ ; 298°K ; déplacements chimiques en ppm ; constantes de couplages J en Hz) : 1,31 (s large, 9H) ; 3,55 (s, 3H) ; 4,14 (d, J = 7,7, 1H); 4,71 (dd, 1H); 5,65 (s large, 1H); 7,18 (d, J = 7, 1H); 7,15-7,3 (m, 5H).

### EXEMPLE 2

Dans un ballon monocol de 5 cm3 muni d'un système d'agitation magnétique, on met 9 mg (0,028 mmole) d'acide benzoyl-3 diméthyl-2,2 phényl-4 oxazolidine-1,3 carboxylique-5-(4S,5S) en solution dans 0,46 cm3 de toluène anhydre. On ajoute ensuite 5,7 mg (0,028 mmole) de dicyclohexylcarbodiimide. On laisse réagir le mélange réactionnel devenu trouble pendant 5 minutes à une température voisine de 20°C puis on ajoute un mélange de 6,4 mg (0,009 mmole) de de diacétoxy-4,10β benzoyloxy-2α époxy-5β,20 dihydroxy-1,13α oxo-9 triéthylsilyloxy-7β taxène-11 et de 1,1 mg (0,009 mmole) de N,N-diméthylamino-4 pyridine. On laisse réagir le mélange réactionnel pendant 5 minutes à une température voisine de 20°C puis on le chauffe pendant 16 heures à 72°C.

Après refroidissement à une température voisine de 20°C, on dilue le mélange réactionnel par addition de 40 cm3 d'acétate d'éthyle. La phase organique est lavée par 2 fois 5 cm3 d'une solution aqueuse saturée de bicarbonate de sodium, 3 fois 5 cm3 d'eau puis 1 fois par 5 cm3 d'une solution aqueuse saturée de chlorure de sodium et enfin séchée sur sulfate de sodium anhydre. Après filtration et élimination des solvants sous pression réduite, le résidu obtenu (21 mg) est purifié par chromatographie sur couche mince de silice en éluant avec un mélange éther éthylique-dichlorométhane (8-92 en volumes) en effectuant deux passages. On obtient ainsi, avec un rendement de 91 %, 8,4 mg (0,008 mmole) de benzoyl-3 diméthyl-2,2 phényl-4 oxazolidine-1,3-carboxylate-5-(4S,5R) de diacétoxy-4,10β benzoyloxy-2α époxy-5β,20 hydroxy-1 oxo-9 triéthylsilyloxy-7β taxène-11 yle-13α dont les caractéristiques sont les suivantes :
- spectre infra-rouge (film) : principales bandes d'absorption caractéristiques à 3400, 2930, 2850, 1730, 1720, 1630, 1590, 1570, 1440, 1360, 1340, 1230, 1195, 1065, 1015, 1005, 980 et 810 cm⁻¹.
- spectre de résonnance magnétique nucléaire du proton (400 MHz ; CDCl₃ ; déplacements chimiques en ppm ; constantes de couplage J en Hz) : 0,54-0,61 (m, 6H) ; 0,92 (t, J = 8, 9H) ; 1,20 (s, 3H) ; 1,22 (s, 3H) ;1,65 (s, 3H) ; 1,86 (s, 3H) ; 1,93 (s large, 3H) ; 2,00 (s, 3H) ; 2,08 (s, 3H) ; 2,19 (s, 3H) ; 1,82-2,15 (m, 3H) ; 2,46-2,54 (m, 1H); 3,77 (d, J = 7,2, 1H); 4,16 (ABq, J_{AB} = 8,4, δ_{A}-δ_{B} = 59,4, 2H) ; 4,46 (dd, J = 6,6 et 10,5, 1H); 4,56 (d, J = 6,8, 1H); 4,88 (d, J = 8, 1H); 5,27 (d, J = 6, 1H); 5,64 (d, J = 7,2, 1H); 6,24 (t, J = 9, 1H); 6,45 (s, 2H) ; 6,94 (s large, 2H aromatiques) ; 7,11-7,26 (m, 8H aromatiques) ; 7,44-7,48 (m, 2H aromatiques) ; 7,59-7,61 (m, 1H aromatique) ; 8,00-8,02 (m, 2H aromatiques)
- spectre de résonance magnétique du ¹³C (100 MHz ; CDCl₃) : 5,20 ; 6,69 ; 9,99 ; 14,26 ; 20,82 ; 21,07 ; 21,62; 26,42 ; 35,27 ; 37,04 ; 43,18 ; 46,69 ; 58,28 ; 65,97 ; 71,68 ; 72,06 ; 74,79 ; 74,85 ; 76,32 ; 78,93 ; 80,74 ; 84,09 ; 93,43 ; 102,65; 126,11; 126,86 ; 127,83 ; 128,02 ; 128,50 ; 128,69 ; 129,14 ; 129,43 ; 130,00 ; 133,67 ; 133,82 ; 138,81 ; 139,90 ; 166,93 ; 169,13 ; 169,85 ; 201,60 - spectre de masse (FAB(+)-matrice NBA+KCl) : 1046, 1008 (MH⁺), 948,930.

L'acide benzoyl-3 diméthyl-2,2 phényl-4 oxazolidine-1,3 carboxylique-5-(4S,5S) peut être préparé de la manière suivante :

Dans un ballon monocol de 2 cm3 muni d'un système d'agitation magnétique, on met 12,5 mg (0,04 mmole) de vinyl-5 benzoyl-3 diméthyl-2,2 phényl-4 oxazolidine-1,3-(4S,5R) en solution dans 80 µl d'acétonitrile. On ajoute ensuite successivement 80 µl de tétrachlorure de carbone, 120 µl d'eau et 22 mg (0,26 mmole) de bicarbonate de sodium pur. On ajoute ensuite, sous agitation vigoureuse et par petites portions, 47 mg (0,22 mmole) de periodate de sodium. On laisse réagir pendant 5 minutes à une température voisine de 20°C, puis on ajoute, en une seule fois, 2,4 mg de trichlorure de ruthénium. Le mélange hétérogène noir résultant est agité vigoureusement à 20°C pendant 72 heures.

Le mélange réactionnel est dilué dans 10 cm3 d'eau. La phase organique basique obtenue est lavée par 3 fois 10 cm3 d'éther. La phase aqueuse basique est refroidie à 0°C, puis elle est acidifiée, sous agitation vigoureuse et en présence de 20cm3 de dichlorométhane, par une solution aqueuse d'acide chlorhydrique 2M jusqu'à pH = 1. Après décantation, la phase aqueuse acide est extraite par 6 fois 15 cm3 de dichlorométhane. Les phases organiques réunies sont lavées 3 fois avec 5 cm3 d'eau puis 1 fois avec 5 cm3 d'une solution aqueuse saturée de chlorure de sodium. Après séchage sur sulfate de sodium anhydre et filtration, la phase organique est concentrée à sec sous pression réduite. On obtient ainsi, avec un rendement de 77 %, 10,0 mg (0,031 mmole) d"acide benzoyl-3 diméthyl-2,2 phényl-4 oxazolidine-1,3 carboxylique-5-(4S,5S) dont les caractéristiques sont les suivantes :
- spectre infra-rouge (film) : principales bandes d'absorption caractéristiques à 3700-2300, 2970, 2940, 2930, 2900, 2825, 1740, 1600, 1590, 1570, 1420-1400, 1370, 1360, 1190, 1180, 1150, 1120, 1090 et 855 cm⁻¹.
- spectre de résonance magnétique nucléaire du proton : (200 MHz ; CDCl₃ ; déplacements chimiques en ppm) : 1,81 (s,3H) ; 2,11 (s, 3H) ; 4,90-5,06 (m, 2H) ; 6,78-6,93 (m, 4H aromatiques) ; 7,07-7,30 (m, 6H aromatiques)

Le vinyl-5 benzoyl-3 diméthyl-2,2 phényl-4 oxazolidine-1,3-(4S,5R) peut être préparée de la manière suivante :

Dans un ballon monocol de 10 cm3, muni d'un système d'agitation magnétique et d'un réfrigérant, on met, sous atmosphère d'argon, 32 mg (0,12 mmole) de phényl-1 benzoylamino-1 hydroxy-2 butène-3-(1S,2R) en suspension dans 0,64 cm3 de toluène anhydre. On ajoute ensuite 226 µl (173 mg , 2,4 mmoles) de méthoxy-2 propène, 6,0 mg (0,024 mmole) de p.toluènesulfonate de pyridinium et 8 grains de tamis moléculaire 4Å. On laisse réagir le mélange réactionnel résultant à une température voisine de 15°C pendant 2,5 heures puis on le chauffe à 100°C pendant 2 heures. Après refroidissement à une température voisine de 15°C, on dilue le mélange réactionnel dans 40 cm3 de dichlorométhane. La phase organique est lavée 1 fois avec 5 cm3 d'une solution saturée de bicarbonate de sodium, 3 fois avec 5 cm3 d'eau et 1 fois avec une solution aqueuse saturée de chlorure de sodiumpuis elle est séchée sur sulfate de sodium anhydre. Après filtration et concentration à sec sous pression réduite, le résidu obtenu est purifié par chromatographie sur gel de silice en éluant une première fois avec un mélange éther éthylique-dichlorométhane (2-98 en volumes) puis une seconde fois avec un mélange acétate d'éthyle-hexane (10-90 en volumes). On obtient ainsi, avec un rendement de 38 %, 14 mg (0,0456 mmole) de vinyl-5 benzoyl-3 diméthyl-2,2 phényl-4 oxazolidine-1,3-(4S,5R) dont les caractéristiques sont les suivantes :
- spectre infra-rouge (film) : principales bandes d'absorption caractéristiques à 3050, 3010, 2980, 2920, 1635, 1595, 1570, 1490, 1385, 1370, 1355, 1245, 1215, 1145, 1065, 1030, 1020, 935, 850 et 690 cm⁻¹.
- spectre de résonance magnétique nucléaire du proton (400 MHz ; CDCl₃ ; déplacements chimiques en ppm ; constantes de couplage J en Hz) : 1,78 (s, 3H) ; 2,01 (s, 3H) ; 4,59 (d, J = 6,4, 1H); 4,79 ( pst, J = 6,4, 1H); 4,97-5,10 (m, 2H) ; 5,21-5,26 (m, 1H); 6,78-6,94 (m, 4H aromatiques) ; 7,04-7,30 (m, 6H aromatiques).

### EXEMPLE 3

Dans un ballon monocol de 5 cm3, muni d'un système d'agitation magnétique, on introduit, sous atmosphère d'argon, 23 mg (0,058 mmole) d'acide t.butoxycarbonyl-3 (méthoxy-4 phényl)-2 phényl-4 oxazolidine-1,3 carboxylique-5-(2R,4S,5S) dans 1 cm3 de toluène anhydre puis on ajoute 11,9 mg (0,058 mmole) de dicyclohexylcarbodiimide. On laisse réagir pendant 5 minutes à une température voisine de 25°C puis on ajoute un mélange de 2,3 mg (0,019 mmole) de N,N-diméthylamino-4 pyridine et de 17 mg (0,019 mmole) d'acétoxy-4 benzoyloxy-2α époxy-5β,20 dihydroxy-1,13α oxo-9 bis-(trichloro-2,2,2 éthoxycarbonyloxy)-7β,10β taxène-11. On laisse réagir pendant 5 minutes à 25°C puis on chauffe pendant 24 heures à 74°C. Après évaporation du toluène sous pression réduite, le résidu obtenu (74 mg) est purifié par chromatographie sur couche mince de gel de silice en éluant avec un mélange éther éthylique-dichlorométhane (5-95 en volumes). On obtient ainsi 23,4 mg (0,012 mmole) de t.butoxycarbonyl-3 (méthoxy-4 phényl)-2 phényl-4 oxazolidine-1,3-carboxylate-5-(2R,4S,5R) d'acétoxy-4 benzoyloxy-2α époxy-5β,20 hydroxy-1 oxo-9 bis-(trichloro-2,2,2 éthoxycarbonyl)oxy -7β,10β taxène-11 yle-13α contaminé par 15 % de t.butoxycarbonyl-3 (méthoxy-4 phényl)-2 phényl-4 oxazolidine-1,3-carboxylate-5-(2R,4S,5S) d'acétoxy-4 benzoyloxy-2α époxy-5β,20 hydroxy-1 oxo-9 bis-(trichloro-2,2,2 éthoxycarbonyl)oxy-7β,10βtaxène-11 yle-13α.

Le t.butoxycarbonyl-3 (méthoxy-4 phényl)-2 phényl-4 oxazolidine-1,3-carboxylate-5-(2R,4S,5R) d'acétoxy-4 benzoyloxy-2α époxy-5β,20 hydroxy-1 oxo-9 bis-(trichloro-2,2,2 éthoxycarbonyl)oxy-7β,10β taxène-11 yle-13α présente les caractéristiques suivantes:
- point de fusion : 164-167°C.
- spectre infra-rouge (film) : principales bandes d'absorption caractéristiques à 3500, 2950, 2900, 1760, 1730, 1720, 1700, 1605, 1580, 1505, 1380, 1375, 1360, 1240, 1140, 1060, 815, 760 et 710 cm⁻¹
- spectre de résonance magnétique nucléaire du proton (500 MHz ; CDCl₃ ; déplacements chimiques en ppm ; constantes de couplage J en Hz): 1,05 (s, 9H) ; 1,16 (s, 3H); 1,24 (s, 3H) ; 1,64 (s, 3H); 1,80 (s, 3H) ; 1,85 (s large, 3H) ; 1,98-2,05 (m, 1H); 2,07-2,14 (m, 1H); 2,18-2,26 (m, 1H); 2,53-2,64 (m, 1H); 3,81 (d, J=7,0, 1H); 3,82 (s, 3H) ; 4,18 (AB_{q}, J_{AB} = 8,5, δ_{A}-δ_{B} = 80,7, 2H) ; 4,58 (s, 1H); 4,74 (AB_{q}, J_{AB} = 11,8, δ_{A}-δ_{B} = 150,6, 2H) ;4,77 (AB_{q}, J_{AB} = 11,8, δ_{A}-δ_{B} = 7,7, 2H) ; 4,88 (d déformé, J = 9,3, 1H); 5,41 (s large, 1H); 5,50 (dd, J = 7,2 et 10,7, 1H); 5,64 (d, J = 7,0, 1H); 6,10 (t, J = 8,8, 1H); 6,14 (s, 1H); 6,40 (s large, 1H); 6,93 (d, J = 8,8, 2H aromatiques) ; 7,26-7,44 (m, 7H aromatiques) ; 7,48-7,52 (m, 2H aromatiques) ; 7,62-7,65 (m, 1H aromatique) ; 8,01-8,03 (m, 2H aromatiques).

Dans un monocol de 5 cm3 muni d'un système d'agitation magnétique, on introduit 13 mg (0,01 mmole) de l'ester obtenu précédemment (mélange des 2 épimères) en solution dans 0,75 cm3 de méthanol puis on ajoute 0,75 cm3 d'acide acétique glacial. On chauffe à 65°C pendant 5 minutes puis on ajoute 65 mg du couple zinc-cuivre (préparé à partir de 20 g de zinc et de 3 g de sulfate de cuivre monohydrate). On agite le mélange hétérogène noir à 65°C pendant 30 minutes. Après refroidissement à une température voisine de 25°C, on dilue le mélange réactionnel dans 30 cm3 de dichlorométhane. On filtre sur célite puis on lave les solides 3 fois avec 10 cm3 de dichlorométhane. On élimine les solvants sous pression réduite. Le résidu obtenu est purifié par chromatographie sur couche mince de gel de silice en éluant avec un mélange méthanol-dichlorométhane (5-95 en volumes). On obtient ainsi, avec un rendement de 60 %, 5,6 mg (0,006 mmole) de t.butoxycarbonyl-3 (méthoxy-4 phényl)-2 phényl-4 oxazolidine-1,3-carboxylate-5-(2R,4S,5R) d'acétoxy-4 benzoyloxy-2α époxy-5β,20 oxo-9 trihydroxy-1,7β,10β taxène-11 yle-13α contaminé par 15-20 % de t.butoxycarbonyl-3 (méthoxy-4 phényl)-2 phényl-4 oxazolidine-1,3-carboxylate-5-(2R,4S,5S) d'acétoxy-4 benzoyloxy-2α époxy-5β,20 oxo-9 trihydroxy-1,7β,10β taxène-11 yle-13α.

Le t.butoxycarbonyl-3 (méthoxy-4 phényl)-2 phényl-4 oxazolidine-1,3-carboxylate-5-(2R,4S,5R) d'acétoxy-4 benzoyloxy-2α époxy-5β,20 oxo-9 trihydroxy-1,7β,10β taxène-11 yle-13α présente les caractéristiques suivantes :
- spectre infra-rouge (film) : bandes d'absorption caractéristiques à 3430, 2960, 2880, 2840, 1730, 1720, 1700, 1685, 1605, 1580, 1505, 1440, 1380, 1360, 1340, 1265, 1240, 1170, 1130, 1060, 1015, 975, 905, 720 et 695 cm⁻¹
- spectre de résonance magnétique nucléaire du proton (400 MHz ; CDCl₃ ;déplacements chimiques en ppm ; constantes de couplage J en Hz) :1,05 (s, 9H) ; 1,09 (s, 3H) ; 1,20 (s, 3H); 1,57 (s, 3H) ; 1,70 (s, 3H) ; 1,73-1,90 (m, 1H); 1,85 (s large, 3H) ; 2,02-2,19 (m, 2H) ; 2,47-2,60 (m, 1H); 3,81 (d, J = 7, 1H); 3,82 (s, 3H) ; 4,15 (d, J = 1,5, 1H); 4,18 (ABq, J_{AB} = 8,5, δ_{A}-δ_{B} = 55,6, 2H) ; 4,56 (d, J = 5,0, 1H); 4,87 (dd déf., J = 8, 1H); 5,10 (d, J = 1,5, 1H); 5,42 (s large, 1H); 5,62 (d, J = 4, 1H) ; 6,13 (t, J = 8, 1H); 6,39 (s large, 1H) ; 6,92 (m, 2H aromatiques) ; 7,30-7,44 (m, 7H aromatiques) ;7,47-7,51 (m, 2H aromatiques) ; 7,59-7,64 (m, 1H aromatique) ; 8,01-8,05 (m, 2H aromatiques)
- spectre de masse (FAB (+) - matrice NBA+KCl) : 1276 (M⁺)
- analyse élémentaire (C₅₇H₆₁O₁₉NCl₆) :

| | | | |
|---|---|---|---|
| calculé | C % 53,62 | H % 4,81 | N % 1,10 |
| trouvé | 53,22 | 4,82 | 1,16 |

Dans un monocol de 5 cm3 muni d'une agitation magnétique, on introduit 4,4 mg (0,0047 mmole) du produit obtenu ci-dessus. On refroidit à 0°C, puis on ajoute 64 µl d'une solution d'acétate d'éthyle contenant 0,28 µl d'acide chlorhydrique à 33 %. On laisse réagir le mélange réactionnel homogène résultant pendant 5 minutes à 0°C puis pendant 5 heures à une température voisine de 25°C. Le mélange réactionnel est dilué dans 20 cm3 d'acétate d'éthyle puis on traite la phase organique par 5 cm3 d'une solution aqueuse saturée de bicarbonate de sodium. La phase organique séparée par décantation est lavée par 3 fois 5 cm3 d'eau et par 1 fois 5 cm3 d'une solution aqueuse saturée de chlorure de sodium puis est séchée sur sulfate de sodium anhydre. Après filtration et concentration à sec sous pression réduite, le résidu obtenu est purifié par chromatographie sur couche mince de gel de silice en éluant avec un mélange méthanol-dichlorométhane (5-95 en volumes). On obtient ainsi, avec un rendement de 78 %, 3,0 mg (0,0037 mmole) de t.butoxycarbonylamino-3 phényl-3 hydroxy-2 propionate-(2R,3S) d'acétoxy-4 benzoyloxy-2α époxy-5β,20 oxo-9 trihydroxy-1,7β,10β taxène-11 yle-13α (ou Taxotere) pur qui ne contient aucune trace de t.butoxycarbonylamino-3 phényl-3 hydroxy-2 propionate-(2S,3S) d'acétoxy-4 benzoyloxy-2α époxy-5β,20 oxo-9 trihydroxy-1,7β,10β taxène-11 yle-13α.

On récupère 0,8 mg (0,0009 mmole) de t.butoxycarbonyl-3 (méthoxy-4 phényl)-2 phényl-4 oxazolidine-1,3-carboxylate-5-(2R,4S,5S) d'acétoxy-4 benzoyloxy-2α époxy-5β,20 oxo-9 trihydroxy-1,7β,10β taxène-11 yle-13α qui n'est pas déprotégé dans les conditions utilisées.

Le Taxotère ainsi obtenu présente les caractéristiques suivantes :
- spectre infra-rouge (film) : principales bandes d'absorption caractéristiques à 3450, 3100, 3050, 2950, 2920, 2890, 2850, 1730, 1710, 1600, 1580, 1490, 1450, 1390, 1370, 1315, 1270, 1245, 1160, 1105, 1095, 1070, 1020, 980, 910, 730 et 710 cm⁻¹.
- spectre de résonance magnétique nucléaire du proton (300 MHz ; CDCl₃ ; déplacements chimiques en ppm ; constantes de couplage J en Hz) : 1,13 (s, 3H); 1,24 (s, 3H) ; 1,34 (s, 9H) ; 1,76 (s, 3H) ; 1,85 (s, 3H) ; 1,74-1,85 (m, 1H); 2,26-2,29 (m, 2H) ; 2,37 (s, 3H) ; 2,54-2,66 (m, 1H); 3,31 (d déformé, J = 4,4, 1H); 3,92 (d, J = 7, 1H); 4,18-4,30 (m, 1H); 4,18 (s, 1H); 4,25 (ABq, J_{AB} = 8,3, δ_{A}-δ_{B} = 35,3, 2H) ; 4,62 (s large, 1H); 4,94 (d, J = 8,5, 1H); 5,20 (s, 1H); 5,26 (s large déformé, 1H); 5,41 (d déformé, J = 9,4, 1H); 5,68 (d, J = 7, 1H) ; 6,21 (t, J = 8,0 et 8,8, 1H); 7,31-7,40 (m, 5H aromatiques) ; 7,47-7,52 (m, 2H aromatiques) ; 7,59-7,64 (m, 1H aromatique) ; 8,09-8,12 (m, 2H aromatiques)

L'acide t.butoxycarbonyl-3 (méthoxy-4 phényl)-2 phényl-4 oxazolidine-1,3 carboxylique-5-(2R,4S,5S) peut être préparé de la manière suivante :

Dans un ballon de 50 cm3 muni d'un système d'agitation magnétique, on met, sous atmosphère d'argon, 33 mg (0,08 mmole) de t.butoxycarbonyl-3 (méthoxy-4 phényl)-2 phényl-4 méthoxycarbonyl-5 oxazolidine-1,3-(2R,4S,5S) en suspension dans 15 cm3 de méthanol. On ajoute ensuite 5 cm3 d'eau et 33 mg (0,24 mmole) de carbonate de potassium. On laisse réagir pendant 96 heures à une température voisine de 25°C. Le mélange réactionnel devient homogène. Le méthanol est éliminé sous pression réduite. La phase aqueuse basique est diluée dans 10 cm3 d'eau. puis on extrait avec 3 fois 15 cm3 d'éther. La phase aqueuse est refroidie à 0°C puis est acidifiée, sous agitation vigoureuseen présence de 20 cm3 de dichlorométhane, par une solution aqueuse d'acide chlorhydrique 4M jusqu'à un pH inférieur à 1. La phase aqueuse acide est extraite 8 fois avec 20 cm3 de dichlorométhane. Les phases organiques réunies sont lavées avec 3 fois 5 cm3 d'eau puis avec 1 fois 5 cm3 d'une solution aqueuse saturée de chlorure de sodium. Les phases organiques sont séchées sur sulfate de sodium anhydre. Après filtration et concentration à sec sous pression réduite, on obtient, avec un rendement de 94 %, 30,0 mg (0,075 mmole) d'acide t.butoxycarbonyl-3 (méthoxy-4 phényl)-2 phényl-4 oxazolidine-1,3 carboxylique-5-(2R,4S,5S) dont les caractéristiques sont les suivantes :
- point de fusion : 148,5-150,5°C.
- pouvoir rotatoire: [α]_{D}²⁵ = + 46,4° (c = 1,0 ; chloroforme)
- spectre infra-rouge (film) : principales bandes d'absorption caractéristiques à 3700-2300, 2950, 2900, 2820, 1755, 1700, 1605, 1580, 1505, 1385, 1360, 1300, 1285, 1240, 1215, 1165, 1130, 1075, 1065, 1020;, 930, 900, 850, 820 et 685 cm⁻¹.
- spectre de résonance magnétique nucléaire du proton sous forme de 2 rotamères (200 MHz ; CDCl₃) ; déplacements chimiques en ppm ; constantes de couplage J en Hz) : 1,11 (s, 9H) ; 3,82 (s, 3H) ; 4,2 (s très large, 1H); 4,99 (d, J = 6,4, 1H); 5,18 (majo d déformé, J = 6,4 ) et 5,36 (mino,s large) (1H); 6,46 (mino) et 6,66 (majo) (s, 2H) ; 6,94 (d, J = 8,6, 2H aromatiques) ; 7,20-7,46 (m, 7H aromatiques).
- spectre de masse (I.C. ; NH₃+ isobutane) : 417 (MH⁺+ NH₃), 400 (MH⁺), 361, 344, 300, 264, 225, 192, 177, 137.

Le t-butoxycarbonyl-3 (méthoxy-4 phényl)-2 phényl-4 méthoxycarbonyl-5 oxazolidine-1,3-(2R,4S,5S) peut être préparé selon l'une des méthodes suivantes :
1) Dans un ballon monocol de 2 cm3, muni d'un système d'agitation magnétique, on met, sous atmosphère d'argon, 10 mg (0,034 mmole) de t.butoxycarbonyl-3 phényl-3 hydroxy-2 propionate-(2S,3S) de méthyle en suspension dans 0,5 cm3 de toluène anhydre. On ajoute ensuite 1 mg (0,004 mmole) de p.toluènesulfonate de pyridinium. On chauffe le mélange réactionel résultant à 115°C. Après 5 minutes à cette température, on ajoute 13 µl (13,9 mg , 0,076 mmole) de p.méthoxybenzaldéhyde diméthylacétal. Le mélange réactionnel devenu homogène est chauffé au reflux du solvant pendant 5 minutes. Après refroidissement à une température voisine de 20°C, on dilue le mélange réactionnel dans 30 cm3 de dichlorométhane. La phase organique est traitée 1 fois par 5 cm3 d'une solution aqueuse saturée de bicarbonate de sodium, puis lavée par 2 fois 5 cm3 d'eau et 1 fois par 5 cm3 d'une solution aqueuse saturée de chlorure de sodium. Après séchage sur sulfate de sodium anhydre, filtration et concentration à sec sous pression réduite, le résidu est purifié par chromatographie sur couche mince de gel de silice en éluant avec un mélange éther éthylique-hexane (6-4 en volumes). On obtient ainsi 13,9 mg (0,0336 mmole) d'un mélange de t.butoxycarbonyl-3 (méthoxy-4 phényl)-2 phényl-4 méthoxycarbonyl-5 oxazolidine-1,3-(2R,4S,5S) et de t.butoxycarbonyl-3 (méthoxy-4 phényl)-2 phényl-4 méthoxycarbonyl-5 oxazolidine-1,3-(2S,4S,5S) dans le rapport 40/60. Le rendement global est de 99 %.
   Ces esters peuvent être séparés par chromatographie sur colonne de gel de silice en éluant avec un mélange éther éthylique-hexane (2-8 en volumes).
   Le t.butoxycarbonyl-3 (méthoxy-4 phényl)-2 phényl-4 méthoxycarbonyl-5 oxazolidine-1,3-(2R,4S,5S) présente les caractéristiques suivantes :
   - point de fusion : 208-208,5°C.
   - pouvoir rotatoire [α]_{D}²⁵ = + 53° (c = 1,1 ; chloroforme)
   - spectre infra-rouge (film) : 2950, 1725, 1680, 1600, 1575, 1500, 1380, 1350, 1280, 1260, 1240, 1200, 1160, 1120, 1065, 1050, 1030 et 1010 cm⁻¹.
   - spectre de résonance magnétique nucléaire du proton sous forme de 2 rotamères (200MHz ; CDCl3 ; déplacements chimiques en ppm ; constantes de couplage J en Hz) : 1,12 (s, 9H) ; 3,32 (s, 3H) ; 3,82 (s, 3H) ; 5,00 (d, J = 6,5, 1H); 5,16 (majo; d déformé, J = 5,6) et 5,34 (mino ; s large, 1H); 6,48 (mino) et 6,68 (majo) (2s, 1H); 6,93 (d, J = 8,4, 2H aromatiques) ; 7,20-7,50 (m, 7 H aromatiques)
   - spectre de masse (D/IC, NH₃ + isobutane) : 414 (MH⁺), 356, 314, 312, 250, 222, 206, 179, 177, 162, 151, 134, 119.

   Le t.butoxycarbonyl-3 (méthoxy-4 phényl)-2 phényl-4 méthoxycarbonyl-5 oxazolidine-1,3-(2S,4S,5S) présente les caractéristiques suivantes :
   - spectre infra-rouge (film) : principales bandes d'absorption caractéristiques à 2950, 2900, 1760, 1730, 1695, 1600, 1580, 1505, 1450, 1430, 1380, 1360, 1335, 1290, 1240, 1210, 1160, 1150, 1080, 1030, 1020, 920, 810 et 680 cm⁻¹.
   - spectre de résonance magnétique nucléaire du proton (200 MHz ; CDCl₃ ; déplacements chimiques en ppm ; constantes de couplage J en Hz) : 1,26 (s, 9H) ; 3,37 (s, 3H) ; 3,82 (s, 3H) ; 5,01 (d, J = 7,1, 1H); 5,27 (d, J = 7,1, 1H); 6,05 (s, 1H); 6,91 (d, J = 8,4, 2H aromatiques) ; 7,26-7,56 (m, 5H aromatiques) ; 7,49 (d, J = 8,4, 2H aromatiques)
   - spectre de masse (D/IC, NH₃ + isobutane) : 414 (MH⁺), 356, 339, 314, 312, 296, 250, 224, 222, 206, 177, 162, 151, 135, 121
2) Dans un ballon de 2 cm3 muni d'un système d'agitation magnétique, on met, sous atmosphère d'argon, 5,0 mg (0,017 mmole) de t.butoxycarbonylamino-3 phényl-3 hydroxy-2 propionate-(2S,3S) de méthyle en suspension dans 0,25 cm3 de toluène anhydre. On ajoute ensuite 10,0 µl (10,7 mg ; 0,059 mmole) de p.méthoxybenzaldéhyde diméthylacétal. On chauffe le mélange réactionnel résultant à 95°C puis on ajoute 1 mg de p.toluènesulfonate de pyridinium-polymère. On poursuit le chauffage pendant 24 heures à 95°C. Après refroidissement à une température voisine de 20°C, on dilue le mélange réactionnel dans 30 cm3 de dichlorométhane. La phase organique est traitée 1 fois par 5 cm3 d'une solution aqueuse saturée de bicarbonate de sodium, puis lavée par 3 fois par 5 cm3 d'eau et 1 fois par 5 cm3 d'une solution aqueuse saturée de chlorure de sodium. Après séchage sur sulfate de sodium anhydre, filtration et concentration à sec, on obtient, après purification par chromatographie sur couche mince de gel de silice en éluant avec un mélange éther éthylique-hexane (1-1 en volumes, 2 passages), avec un rendement de 93 %, 6,5 mg (0,016 mmole) d'un mélange de t.butoxycarbonyl-3 (méthoxy-4 phényl)-2 phényl-4 méthoxycarbonyl-5 oxazolidine-1,3-(2R,4S,5S) et de t.butoxycarbonyl-3 (méthoxy-4 phényl)-2 phényl-4 méthoxycarbonyl-5 oxazolidine-1,3-(2S,4S,5S) dans le rapport 30/70.
3) Dans un ballon monocol de 2 cm3 muni d'un système d'agitation magnétique, on met sous atmosphère d'argon, 10,0 mg (0,034 mmole) de t.butoxycarbonylamino-3 phényl-3 hydroxy-2 propionate-(2S,3S) de méthyle en suspension dans 0,5 cm3 de toluène anhydre. On'ajoute ensuite 13,0 µl (13,9 mg , 0,076 mmole) de p.méthoxybenzaldéhyde diméthylacétal. On chauffe le mélange réactionnel résultant à 74°C pendant 5 minutes puis on ajoute 2,5 mg d'acide p.toluènesulfonique monohydrate. On poursuit le chauffage à 74°C pendant 17 heures. Après refroidissement, le mélange réactionnel est dilué dans 30 cm3 de dichlorométhane. La phase organique est traitée 1 fois par 5 cm3 d'une solution aqueuse saturée de bicarbonate de sodium, puis lavée 2 fois par 5 cm3 d'eau et 1 fois par 5 cm3 d'une solution aqueuse saturée de chlorure de sodium. Après séchage sur sulfate de sodium anhydre, filtration et concentration à sec sous pression réduite, on obtient, après purification par chromatographie sur couche mince de gel de silice en éluant avec un mélange éther éthylique-hexane (1-1 en volumes), avec un rendement de 45%, 6,3 mg (0,015 mmole) d'un mélange de t.butoxycarbonyl-3 (méthoxy-4 phényl)-2 phényl-4 méthoxycarbonyl-5 oxazolidine-1,3-(2R,4S,5S) et de t.butoxycarbonyl-3 (méthoxy-4 phényl)-2 phényl-4 méthoxycarbonyl-5 oxazolidine-1,3-(2S,4S,5S) dans le rapport 61/39.
4) Dans un ballon monocol de 2 cm3 muni d'un système d'agitation magnétique, on met sous atmosphère d'argon, 5,0 mg (0,017 mmole) de t.butoxycarbonylamino-3 phényl-3 hydroxy-2 propionate-(2S,3S) de méthyle en suspension dans 0,25 cm3 de toluène anhydre. On ajoute ensuite 6,5 µl (6,95 mg ; 0,038 mmole) de p.méthoxybenzaldéhyde diméthylacétal. On chauffe le mélange réactionnel résultant à 76°C pendant 5 minutes puis on ajoute 0,5 mg (0,002 mmole) d'acide camphorsulfonique. On poursuit le chauffage à 76°C. Après 4 heures de réaction, on ajoute 4,0 µl (2,43 mg , 0,076 mmole) de méthanol et laisse réagir pendant encore 44 heures à 76°C. Après refroidissement, le mélange réactionnel est dilué dans 30 cm3 de dichlorométhane. La phase organique est traitée 1 fois par 5 cm3 d'une solution aqueuse saturée de bicarbonate de sodium, puis lavée 2 fois par 5 cm3 d'eau et 1 fois par 5 cm3 d'une solution aqueuse saturée de chlorure de sodium. Après séchage sur sulfate de sodium anhydre, filtration et concentration à sec sous pression réduite, on obtient, après purification par chromatographie sur couche mince de gel de silice en éluant avec un mélange éther éthylique-hexane (3-2 en volumes), avec un rendement de 53 %, 3,7 mg (0,009 mmole) d'un mélange de t.butoxycarbonyl-3 (méthoxy-4 phényl)-2 phényl-4 méthoxycarbonyl-5 oxazolidine-1,3-(2R,4S,5S) et de t.butoxycarbonyl-3 (méthoxy-4 phényl)-2 phényl-4 méthoxycarbonyl-5 oxazolidine-1,3-(2S,4S,5S) dans le rapport 74/26.

## Revendications

1. Procédé de préparation de dérivés du taxane de formule générale : 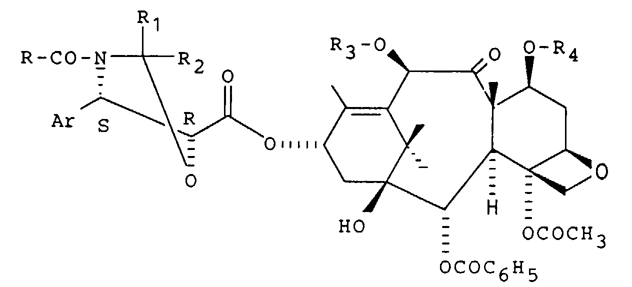 dans laquelle
Ar représente un radical aryle,
R représente le radical phényle ou un radical R₅-O- dans lequel R₅ représente
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle cyano, carboxy ou alcoxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone,
- un radical hétérocyclyle azoté saturé ou non saturé contenant 4 à 6 chaînons et éventuellement substitué par un ou plusieurs radicaux alcoyles contenant 1 à 4 atomes de carbone, étant entendu que les radicaux cycloalcoyles, cycloalcényles ou bicycloalcoyles peuvent être éventuellement substitués par un ou plusieurs radicaux alcoyles contenant 1 à 4 atomes de carbone,
R₁ et R₂, identiques ou différents, représentent un atome d'hydrogène, un radical alcoyle, phénylalcoyle, phényle, alcoxyphényle ou dialcoxyphényle, ou bien R1 et R2 forment ensemble avec l'atome de carbone auquel ils sont liés un cycle ayant de 4 à 7 chaînons,
R₃ représente un radical acétyle ou un groupement protecteur de la fonction hydroxy et
R₄ représente un groupement protecteur de la fonction hydroxy caractérisé en ce que l'on estérifie la baccatine III protégée ou la 10-désacétylbaccatine III protégée de formule générale : 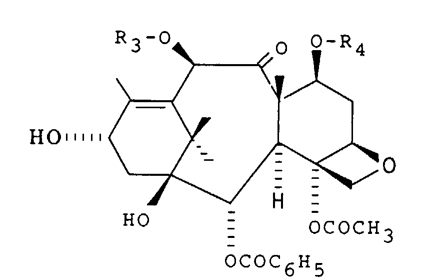 dans laquelle R₃ et R₄ sont définis comme précédemment au moyen d'un acide de formule générale : 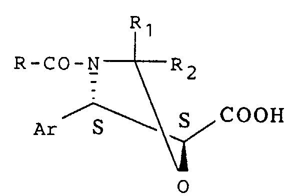 dans laquelle Ar, R, R₁ et R₂ sont définis comme précédemment, ou d'un dérivé activé de cet acide.

2. Procédé selon la revendication 1 caractérisé en ce que l'estérification est effectuée au moyen d'un acide de formule générale : 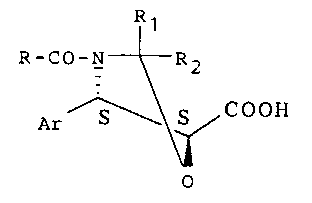 dans laquelle Ar, R, R₁ et R₂ sont définis comme dans la revendication 1 en opérant en présence d'un agent de condensation et d'un agent d'activation dans un solvant organique à une température comprise entre 0 et 90°C.

3. Procédé selon la revendication 2 caractérisé en ce que l'agent de condensation est choisi parmi les imides et les carbonates réactifs et l'agent d'activation est choisi parmi les aminopyridines.

4. Procédé selon la revendication 3 caractérisé en ce que l'agent de condensation est choisi parmi le dicyclohexylcarbodiimide et le 2-dipyridylcarbonate et l'agent d'activation est choisi parmi la 4-diméthylaminopyridine ou la 4-pyrrolidinopyridine.

5. Procédé selon la revendication 2 caractérisé en ce que le solvant est choisi parmi les éthers, les cétones, les esters, les nitriles, les hydrocarbures aliphatiques, les hydrocarbures aliphatiques halogénés et les hydrocarbures aromatiques.

6. Procédé selon la revendication 5 caractérisé en ce que le solvant est choisi parmi les hydrocarbures aromatiques.

7. Procédé selon la revendication 1 caractérisé en ce que l'estérification est effectuée au moyen d'un anhydride de formule générale : 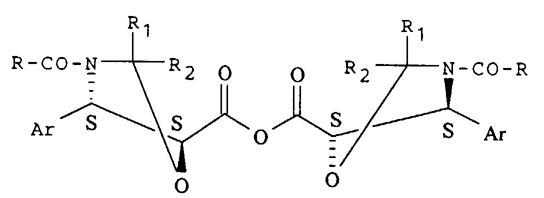 dans laquelle Ar, R, R₁ et R₂ sont définis comme dans la revendication 1 en opérant en présence d'un agent d'activation dans un solvant organique à une température comprise entre 0 et 90°C.

8. Procédé selon la revendication 7 caractérisé en ce que l'agent d'activation est choisi parmi les aminopyridines.

9. Procédé selon la revendication 8 caractérisé en ce que l'agent d'activation est choisi parmi la 4-diméthylaminopyridine ou la 4-pyrrolidinopyridine.

10. Procédé selon la revendication 7 caractérisé en ce que le solvant est choisi parmi les éthers, les cétones, les esters, les nitriles, les hydrocarbures aliphatiques, les hydrocarbures aliphatiques halogénés et les hydrocarbures aromatiques.

11. Procédé selon la revendication 1 caractérisé en ce que l'estérification est effectuée au moyen d'un acide activé de formule générale : 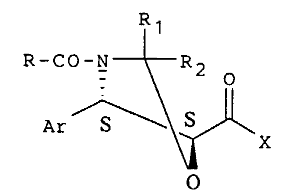 dans laquelle Ar, R, R₁ et R₂ sont définis comme dans la revendication 1 et X représente un atome d'halogène ou un radical acyloxy ou aroyloxy, éventuellement préparé in situ en présence d'une base en opérant dans un solvant organique à une température comprise entre 0 et 90°C.

12. Procédé selon la revendication 11 caractérisé en ce que la base est choisie parmi les bases organiques azotées.

13. Procédé selon la revendication 12 caractérisé en ce que la base organique azotée est choisie parmi les amines tertiaires aliphatiques, la pyridine et les aminopyridines.

14. Procédé selon la revendication 11 caractérisé en ce que le solvant organique est choisi parmi les éthers, les cétones, les esters, les nitriles, les hydrocarbures aliphatiques, les hydrocarbures aliphatiques halogénés et les hydrocarbures aromatiques.

15. Procédé selon la revendication 14 caractérisé en ce que le solvant est choisi parmi les hydrocarbures aromatiques.

16. Les acides de formule générale : 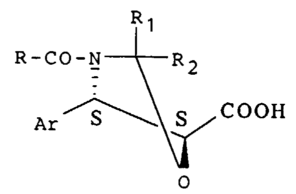 dans laquelle Ar, R, R₁ et R₂ sont définis comme dans la revendication 1, éventuellement sous forme de sel, d'ester, d'anhydride, d'anhydride mixte ou d'halogénure.

17. Dérivés de formule générale : 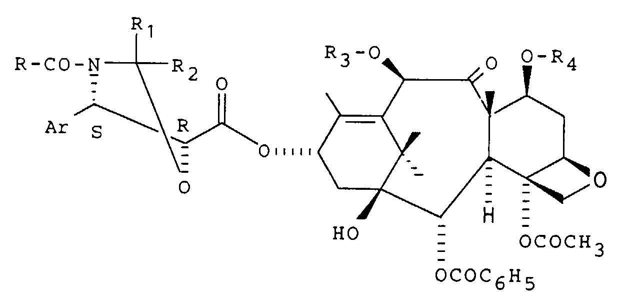 dans laquelle
Ar représente un radical aryle,
R représente le radical phényle
R₁ et R₂, identiques ou différents, représentent un atome d'hydrogène, un radical alcoyle, phénylalcoyle, phényle, alcoxyphényle ou dialcoxyphényle, ou bien R1 et R2 forment ensemble avec l'atome de carbone auquel ils sont liés un cycle ayant de 4 à 7 chaînons, étant entendu que, lorsque l'un des R₁ ou R₂ représente un atome d'hydrogène, alors l'autre des R₁ ou R₂ ne représente ni un atome d'hydrogène, ni un radical aryle éventuellement substitué,
R₃ représente un radical acétyle ou un groupement protecteur de la fonction hydroxy et
R₄ représente un groupement protecteur de la fonction hydroxy.

## Patentansprüche

1. Verfahren zur Herstellung von Taxanderivaten der allgemeinen Formel 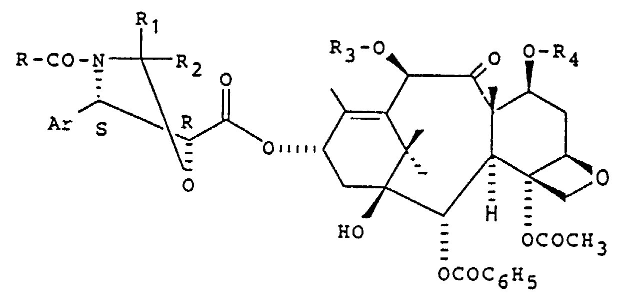 worin
Ar einen Arylrest bedeutet,
R einen Phenylrest oder einen Rest R₅-D bedeutet, worin R₅
- einen geradkettigen oder verzweigten Alkylrest mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 11 Kohlenstoffatomen, wobei diese Reste gegebenenfalls substituiert sind durch einen oder mehrere Substituenten, ausgewählt unter den Halogenatomen und den Hydroxyresten, Alkoxy mit 1 bis 4 Kohlenstoffatomen, Dialkylamino, dessen Alkylteil 1 bis 4 Kohlenstoffatome aufweist, Piperidino, Morpholino, Piperazin-1-yl (gegebenenfalls substituiert in 4-Stellung durch einen Alkylrest mit 1 bis 4 Kohlenstoffatomen oder durch einen Phenylalkylrest, dessen Alkylteil 1 bis 4 Kohlenstoffatome aufweist), Cycloalkyl mit 3 bis 6 Kohlenstoffatomen, Cycloalkenyl mit 4 bis 6 Kohlenstoffatomen, Phenylcyano, Carboxy oder Alkoxycarbonyl, dessen Alkylteil 1 bis 4 Kohlenstoffatome aufweist,
- oder einen Phenylrest, gegebenenfalls substituiert durch ein oder mehrere Atome oder Reste,ausgewählt unter den Halogenatomen und den Alkylresten mit 1 bis 4 Kohlenstoffatomen oder Alkoxyresten mit 1 bis 4 Kohlenstoffatomen,
- einen gesättigten oder ungesättigten, stickstoffhaltigen Heterocyclylrest mit 4 bis 6 Gliedern und gegebenenfalls substituiert durch einen oder mehrere Alkylreste mit 1 bis 4 Kohlenstoffatomen, wobei die Cycloalkyl-, Cycloalkenyl- oder Bicycloalkylreste gegebenenfalls durch einen oder mehrere Alkylreste mit 1 bis 4 Kohlenstoffatomen substituiert sein können,
wiedergibt,
R₁ und R₂, identisch oder verschieden, ein Wasserstoffatom, einen Alkyl-, Phenylalkyl-, Phenyl-, Alkoxyphenyl- oder Dialkoxyphenylrest bedeuten oder R₁ und R₂ gemeinsam mit dem Kohlenstoffatom, an das sie gebunden sind, einen Ring mit 4 bis 7 Gliedern bilden,
R₃ einen Acetylrest oder eine Schutzgruppe für die Hydroxyfunktion darstellt und
R₄ eine Schutzgruppe für die Hydroxyfunktion wiedergibt,
dadurch gekennzeichnet, daß man geschütztes Baccatin III oder geschütztes 10-Desacetylbaccatin III der allgemeinen Formel 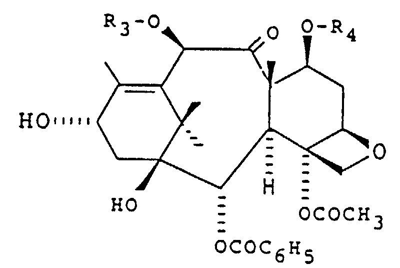 worin R₃ und R₄ wie vorstehend definiert sind, mit einer Säure der allgemeinen Formel 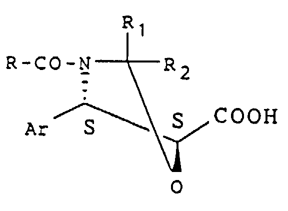 worin Ar, R, R₁ und R₂ wie vorstehend definiert sind, oder einem aktiven Derivat dieser Säure verestert.

2. Verfahren gemäß Anspruch 1, dadurch gekennzeichnet, daß die Veresterung mit Hilfe einer Säure der allgemeinen Formel 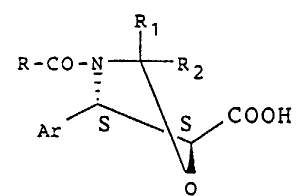 worin Ar, R, R₁ und R₂ wie in Anspruch 1 definiert sind, durchgeführt wird, wobei man in Anwesenheit eines Kondensationsmittels und eines Aktivierungsmittels in einem organischen Lösungsmittel bei einer Temperatur zwischen 0 und 90°C arbeitet.

3. Verfahren gemäß Anspruch 2, dadurch gekennzeichnet, daß das Kondensationsmittel unter den reaktiven Imiden und Carbonaten und das Aktivierungsmittel unter den Aminopyridinen ausgewählt wird.

4. Verfahren gemäß Anspruch 3, dadurch gekennzeichnet, daß das Kondensationsmittel unter Dicyclohexylcarbodiimid und 2-Dipyridylcarbonat und das Aktivierungsmittel unter 4-Dimethylaminopyridin oder 4-Pyrrolidinopyridin ausgewählt werden.

5. Verfahren gemäß Anspruch 2, dadurch gekennzeichnet, daß das Lösungsmittel unter den Ethern, Ketonen, Estern, Nitrilen, aliphatischen Kohlenwasserstoffen, halogenierten aliphatischen Kohlenwasserstoffen und aromatischen Kohlenwasserstoffen ausgewählt wird.

6. Verfahren gemäß Anspruch 5, dadurch gekennzeichnet, daß das Lösungsmittel unter den aromatischen Kohlenwasserstoffen ausgewählt wird.

7. Verfahren gemäß Anspruch 1, dadurch gekennzeichnet, daß die Veresterung mit Hilfe eines Anhydrids der allgemeinen Formel 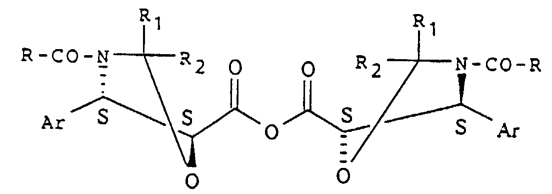 worin Ar, R, R₁ und R₂ wie in Anspruch 1 definiert sind, durchgeführt wird, wobei man in Gegenwart eines Aktivierungsmittels in einem organischen Lösungsmittel bei einer Temperatur zwischen 0 und 90°C arbeitet.

8. Verfahren gemäß Anspruch 7, dadurch gekennzeichnet, daß das Aktivierungsmittel unter den Aminopyridinen ausgewählt wird.

9. Verfahren gemäß Anspruch 8, dadurch gekennzeichnet, daß das Aktivierungsmittel unter 4-Dimethylaminopyridin oder 4-Pyrrolidinopyridin ausgewählt wird.

10. Verfahren gemäß Anspruch 7, dadurch gekennzeichnet, daß das Lösungsmittel unter den Ethern, Ketonen, Estern, Nitrilen, aliphatischen Kohlenwasserstoffen, halogenierten aliphatischen Kohlenwasserstoffen und aromatischen Kohlenwasserstoffen ausgewählt wird.

11. Verfahren gemäß Anspruch 1, dadurch gekennzeichnet, daß die Veresterung mit Hilfe einer aktivierten Säure der allgemeinen Formel 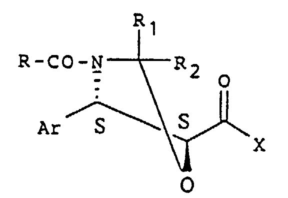 worin Ar, R, R₁ und R₂ wie in Anspruch 1 definiert sind und X ein Halogenatom oder einen Acyloxy- oder Aroyloxyrest bedeutet, gegebenenfalls hergestellt in situ in Anwesenheit einer Base, durchgeführt wird, wobei man in einem organischen Lösungsmittel bei einer Temperatur zwischen 0 und 90°C arbeitet.

12. Verfahren gemäß Anspruch 11, dadurch gekennzeichnet, daß die Base unter den organischen Stickstoffbasen ausgewählt wird.

13. Verfahren gemäß Anspruch 12, dadurch gekennzeichnet, daß die organische Stickstoffbase unter den aliphatischen tertiären Aminen, Pyridin und den Aminopyridinen ausgewählt wird.

14. Verfahren gemäß Anspruch 11, dadurch gekennzeichnet, daß das organische Lösungsmittel unter den Ethern, Ketonen, Estern, Nitrilen, aliphatischen Kohlenwasserstoffen, halogenierten aliphatischen Kohlenwasserstoffen und aromatischen Kohlenwasserstoffen ausgewählt wird.

15. Verfahren gemäß Anspruch 14, dadurch gekennzeichnet, daß das Lösungsmittel unter den aromatischen Kohlenwasserstoffen ausgewählt wird.

16. Die Säuren der allgemeinen Formel 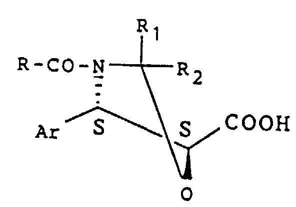 worin Ar, R, R₁ und R₂ wie in Anspruch 1 definiert sind, gegebenenfalls in Form des Salzes, Esters, Anhydrids, gemischten Anhydrids oder Halogenids.

17. Derivate der allgemeinen Formel 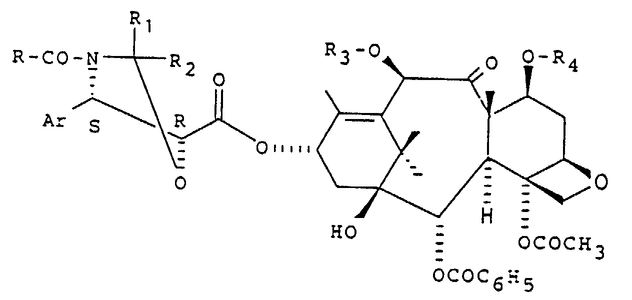 worin
Ar einen Arylrest bedeutet,
R für den Phenylrest steht,
R₁ und R₂, identisch oder verschieden, ein Wasserstoffatom, einen Alkyl-, Phenylalkyl-, Phenyl-, Alkoxyphenyl- oder Dialkoxyphenylrest bedeuten oder R₁ und R₂ gemeinsam mit dem Kohlenstoffatom, an das sie gebunden sind, einen Ring mit 4 bis 7 Gliedern bilden, mit der Maßgabe, daß, wenn eines von R₁ oder R₂ ein Wasserstoffatom bedeutet, das andere von R₁ oder R₂ weder ein Wasserstoffatom noch einen gegebenenfalls substituierten Arylrest darstellt,
R₃ einen Acetylrest oder eine Schutzgruppe für die Hydroxyfunktion bedeutet und
R₄ für eine Schutzgruppe für die Hydroxyfunktion steht.

## Claims

1. Process for the preparation of taxane derivatives of general formula: 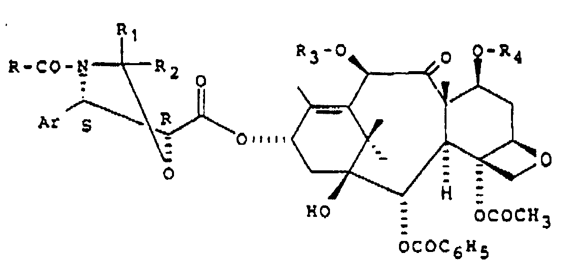 in which
Ar represents an aryl radical,
R represents the phenyl radical or a radical R₆-O- in which R₅ represents
- a straight or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 11 carbon atoms, these radicals optionally being substituted by one or a number of substituents chosen from the halogen atoms and the hydroxyl radical, alkoxy radical containing 1 to 4 carbon atoms, dialkylamino radical, each alkyl part of which contains 1 to 4 carbon atoms, piperidino radical, morpholino radical, 1-piperazinyl radical (optionally substituted in the 4-position by an alkyl radical containing 1 to 4 carbon atoms or by a phenylalkyl radical, the alkyl part of which contains 1 to 4 carbon atoms), cycloalkyl radical containing 3 to 6 carbon atoms, cycloalkenyl radical containing 4 to 6 carbon atoms, phenyl cyano radical, carboxyl radical or alkoxycarbonyl radical, the alkyl part of which contains 1 to 4 carbon atoms,
- or a phenyl radical optionally substituted by one or a number of atoms or radicals chosen from the halogen atoms and the alkyl radicals containing 1 to 4 carbon atoms or the alkoxy radicals containing 1 to 4 carbon atoms,
- a saturated or unsaturated nitrogen-containing heterocyclyl radical containing 4 to 6 members and optionally substituted by one or a number of alkyl radicals containing 1 to 4 carbon atoms, it being understood that the cycloalkyl, cycloalkenyl or bicycloalkyl radicals may optionally be substituted by one or a number of alkyl radicals containing 1 to 4 carbon atoms,
R₁ and R₂, which are identical or different, represent a hydrogen atom or an alkyl, phenylalkyl, phenyl, alkoxyphenyl or dialkoxyphenyl radical or else R₁ and R₂ form, together with the carbon atom to which they are bonded, a ring having from 4 to 7 members,
R₃ represents an acetyl radical or a protective group of the hydroxyl functional group
and
R₄ represents a protective group of the hydroxyl functional group, characterized in that protected baccatin III or protected 10-deacetylbaccatin III of general formula: 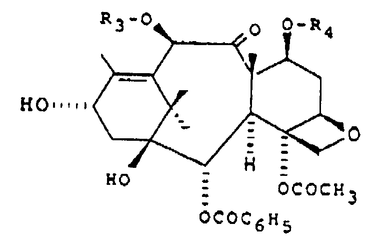 in which R₃ and R₄ are defined as above, is esterified by means of an acid of general formula: 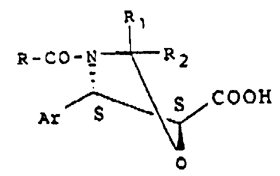 in which Ar, R, R₁ and R₂ are defined as above, or of an activated derivative of this acid.

2. Process according to claim 1, characterized in that the esterification is carried out by means of an acid of general formula: 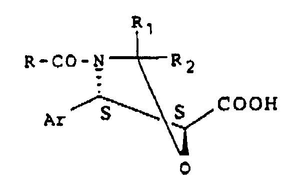 in which Ar, R, R₁ and R₂ are defined as in claim 1, the reaction being carried out in the presence of a condensation agent and of an activating agent in an organic solvent at a temperature between 0 and 90°C.

3. Process according to claim 2, characterized in that the condensation agent is chosen from imides and reactive carbonates and the activating agent is chosen from aminopyridines.

4. Process according to claim 3, characterized in that the condensation agent is chosen from dicyclohexylcarbodiimide and di-2-pyridyl ketone and the activating agent is chosen from 4-dimethylaminopyridine or 4-pyrrolidinopyridine.

5. Process according to claim 2, characterized in that the solvent is chosen from ethers, ketones, esters, nitriles, aliphatic hydrocarbons, halogenated aliphatic hydrocarbons and aromatic hydrocarbons.

6. Process according to claim 5, characterized in that the solvent is chosen from aromatic hydrocarbons.

7. Process according to claim 1, characterized in that the esterification is carried out by means of an anhydride of general formula: 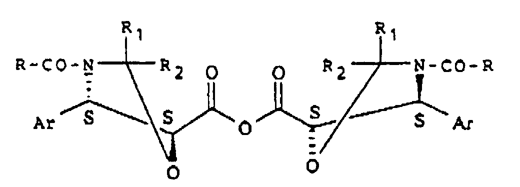 in which Ar, R, R₁ and R₂ are defined as in claim 1, the reaction being carried out in the presence of an activating agent in an organic solvent at a temperature between 0 and 90°C.

8. Process according to claim 7, characterized in that the activating agent is chosen from aminopyridines.

9. Process according to claim 8, characterized in that the activating agent is chosen from 4-dimethylaminopyridine or 4-pyrrolidinopyridine.

10. Process according to claim 7, characterized in that the solvent is chosen from ethers, ketones, esters, nitriles, aliphatic hydrocarbons, halogenated aliphatic hydrocarbons and aromatic hydrocarbons.

11. Process according to claim 1, characterized in that the esterification is carried out by means of an activated acid of general formula: 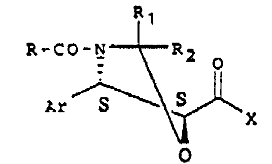 in which Ar, R, R₁ and R₂ are defined as in claim 1 and X represents a halogen atom or an acyloxy or aroyloxy radical, optionally prepared in situ in the presence of a base, the reaction being carried out in an organic solvent at a temperature between 0 and 90°C.

12. Process according to claim 11, characterized in that the base is chosen from nitrogenous organic bases.

13. Process according to claim 12, characterized in that the nitrogenous organic base is chosen from aliphatic tertiary amines, pyridine and aminopyridines.

14. Process according to claim 11, characterized in that the organic solvent is chosen from ethers, ketones, esters, nitriles, aliphatic hydrocarbons, halogenated aliphatic hydrocarbons and aromatic hydrocarbons.

15. Process according to claim 14, characterized in that the solvent is chosen from aromatic hydrocarbons.

16. Acids of general formula: 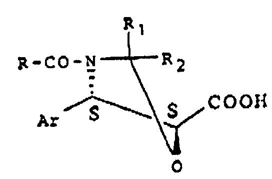 in which Ar, R, R₁ and R₂ are defined as in claim 1, optionally in the salt, ester, anhydride, mixed anhydride or halide form.

17. Derivatives of general formula: 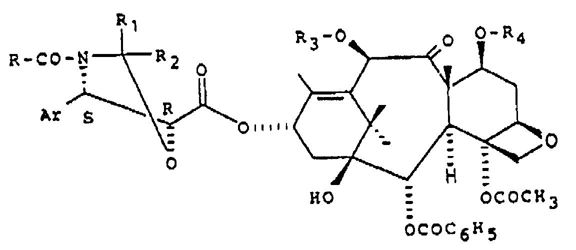 in which
Ar represents an aryl radical,
R represents the phenyl radical,
R₁ and R₂, which are identical or different, represent a hydrogen atom or an alkyl, phenylalkyl, phenyl, alkoxyphenyl or dialkoxyphenyl radical or else R₁ and R₂ form, together with the carbon atom to which they are bonded, a ring having from 4 to 7 members, it being understood that, when one of R₁ or R₂ represents a hydrogen atom, then the other R₁ or R₂ represents neither a hydrogen atom nor an optionally substituted aryl radical,
R₃ represents an acetyl radical or a protective group of the hydroxyl functional group and
R₄ represents a protective group of the hydroxyl functional group.
